# EUROPEAN PATENT APPLICATION

(11) **EP 2 398 245 A2**
(43) Date of publication of application: **21.12.2011**
(21) Application number: 11166362.1
(22) Date of filing: 17.05.2011
(51) Int. Cl.: H04N 13/00

(54) **Display apparatus and 3d image acquisition-examination method thereof**

(30) Priority: 17.06.2010 KR 20100057630
(71) Applicant: SAMSUNG ELECTRONICS CO., LTD., Suwon-si Gyeonggi-do 442-742 (KR)
(72) Inventor: KWRK, Byung-ju, Gyeonggi-do (KR); KIM, Jae-cheol, Gyeonggi-do (KR); LEE, Bong-geun, Gyeonggi-do (KR); SHIN, Jun-hyung, Gyeonggi-do (KR); LEE, Joo-won, Gyeonggi-do (KR)
(74) Representative: Fearnside, Andrew Simon

(57) **Abstract**

A display apparatus providing a three-dimensional (3D) image acquisition-examination mode is disclosed. The display apparatus includes a display unit to display a 3D examination test shape for examining a degree of 3D image acquisition, a user interface unit to allow a user to input a degree of the user's acquisition to the 3D examination test shape, and a control unit controlling to display an output according to the degree of the user's acquisition input through the user interface unit.

## Description

### CROSS-REFERENCE TO RELATED APPLICATION

This application claims priority from Korean Patent Application No. 10-2010-57630, filed June 17, 2010, in the Korean Intellectual Property Office, the disclosure of which is incorporated herein by reference in its entirety.

### BACKGROUND

### 1. Field

Apparatuses and methods consistent with the present invention relate to a display apparatus and a display method thereof, which displays a three dimensional (3D) image.

### 2. Description of the Related Art

A 3D or stereoscopic image technology is being applied in various fields, such as an information and telecommunication, broadcasting, educational training, military affairs, games, animation, virtual reality, computer-aided design (CAD), industrial technologies, and others, and forms a basic core technology of next generation 3D multimedia information and telecommunication systems in the aforementioned fields,

Generally, a stereoscopic effect that a human perceives is the result of a combination of effects, including a degree of change in thickness of a person's eye lens according to a position of an object being observed, a difference in the angle of the object as perceived by the left and right eyes, differences in position and shape of an object as perceived by the left and right eyes, a disparity caused by a movement of the object, and various other psychological and memory effects.

In particular, the binocular disparity, caused by an approximate 6-7 cm lateral distance between a person's left and right eyes, is a very important factor of the stereoscopic effect. In other words, a person perceives an object at different angles due to binocular disparity. Images perceived through the left and right eyes are different due to the difference in the different angles between the left and right eyes and the object. When the two images are transmitted through respective retinas of the two eyes to the brain, the brain unites the two image information together and thus a person perceives a stereoscopic image.

There are two types of stereoscopic image display apparatuses: glasses type display apparatuses using special glasses, and a non-glasses type display apparatuses which do not use the special glasses. Glasses type display apparatuses may be a color filter type display apparatus which divides and selects images by using color filters of complementary colors, a polarization filter type display apparatus which divides left and right eye images using a shading effect caused by a combination of orthogonal polarization elements, or a shutter glasses type display apparatus allowing a user to perceive a 3D effect by alternatively shading left and right eye images in response to synchronized signals for projecting left and right eye image signals on a screen.

As described above, because the 3D image is perceived using binocular vision, it is impossible to properly perceive a 3D image if there is a problem with the binocular vision. Accordingly, there is a need for a method to check binocular vision in advance and if necessary, to cure a problem thereof to maintain a state where the user can perceive the 3D image.

### SUMMARY

Aspects of exemplary embodiments overcome the above disadvantages and other disadvantages not described above. Also, aspects of exemplary embodiments are not required to overcome the disadvantages described above, and aspects of exemplary embodiments may not overcome any of the problems described above.

According to the present invention there is provided an apparatus and method as set forth in the appended claims. Other features of the invention will be apparent from the dependent claims, and the description which follows.

According to an aspect of an exemplary embodiment, there is provided a display apparatus and a 3D image acquisition-examination method thereof, which can examine a degree of 3D image acquisition through self-diagnosis.

According to an aspect of another exemplary embodiment, a display apparatus providing a 3D image acquisition-examination mode includes a display unit to display a 3D examination test shape for examining a degree of 3D image acquisition, a user interface unit to allow a user to input a degree of the user's acquisition to the 3D examination test shape, and a control unit controlling to display an output according to the degree of the user's acquisition input through the user interface unit.

In addition, the control unit may control the display unit to display an informing message including a 3D acquisition-examination result or a guide comment according to the degree of the user's acquisition input through the user interface unit.

Further, the control unit may control to enter the 3D image acquisition-examination mode according to a user command or a preset event input through the user interface unit.

The 3D examination test shape may include at least one of an image capable of being viewed by a left eye, an image capable of being viewed by a right eye, and an image capable of being simultaneously viewed by both the left eye and the right eye.

Furthermore, the control unit may control to display shapes practicable when the 3D examination test shape is incompletely acquired, according to a user command or a preset event input through the user interface unit.

Moreover, the 3D image acquisition-examination mode may include an eyestrain acquisition-examination mode, and the 3D examination test shape may include a predetermined figure form, a midpoint in the figure form capable of being viewed by one of the user's eyes, and a symbol form in the figure form capable of being viewed by the other of the user's eyes.

The 3D image acquisition-examination mode may include a definition acquisition-examination mode, and the 3D examination test shape may include an upper figure form capable of being viewed by one of the user's eyes, a lower figure form positioned below the upper figure form and capable of being viewed by the other of the user's eyes, and a line form positioned between the upper line form and the lower line form and capable of being simultaneously viewed by the both eyes.

Also, the 3D image acquisition-examination mode may include at least one of a coarse and fine examination mode for determining whether the user perceives a three-dimensional effect, a separate heterophoria examination mode for determining whether both eyes are correctly positioned with respect to a horizontal direction, a combined heterophoria examination mode for simultaneously examining a horizontal heterophoria and a vertical heterophoria, an aniseikonia-examination mode for examining whether magnitudes or shapes of retinal images in the left and right eyes are the same, and a binocular fusion examination mode for examining whether there is fusion and suppression at long and short distances.

Also, the 3D image acquisition-examination mode may include at least one of a binocular disparity determination mode for determining a difference between visual powers in the user's eyes, a distance sensibility determination mode for determining an acquisition ability for distance perception, and a latent heterophoria determination mode for determining whether there is latent heterophoria.

Here, the 3D examination test shape may be an image stored in advance or an image received from an external source.

According to an aspect of another exemplary embodiment, a 3D image acquisition-examination method providing a 3D image acquisition-examination mode includes displaying a 3D examination test shape for examining a degree of 3D image acquisition, inputting a degree of a user's acquisition to the 3D examination test shape, and displaying an output according to the input degree of the user's acquisition.

The displaying the output comprises displaying an informing message including a 3D acquisition-examination result or a guide comment according to the input degree of the user's acquisition.

In addition, the method may further include entering the 3D image acquisition-examination mode according to a user command or a preset event.

The 3D examination test shape may include at least one of an image capable of being viewed by a left eye, an image capable of being viewed by a right eye, and an image capable of being simultaneously viewed by both the left eye and the right eye.

Further, the method may further include displaying shapes practicable when the 3D examination test image is incompletely acquired, according to a user command or a preset event.

Furthermore, the 3D image acquisition-examination mode may include an eyestrain acquisition-examination mode, and the 3D examination test shape may include a predetermined figure form, a midpoint in the figure form capable of being viewed by one of the user's eyes, and a symbol form in the figure form capable of being viewed by the other of the user's eyes.

The 3D image acquisition-examination mode may include a focus acquisition-examination mode, and the 3D examination test shape comprises an upper figure capable of being viewed by one of the user's eyes, a lower figure form positioned below the upper figure form and capable of being viewed by the other of the user's eyes, and a line form positioned between the upper figure form and the lower figure form and capable of being simultaneously acquired by both of the user's eyes.

Moreover, the 3D image acquisition-examination mode may include at least one of a coarse and fine examination mode for determining whether the user perceives a three-dimensional effect, a separate heterophoria examination mode for determining whether both eyes are correctly positioned with respect to a horizontal plane, a combined heterophoria examination mode for simultaneously examining a horizontal heterophoria and a vertical heterophoria, an aniseikonia-examination mode for examining whether magnitudes or shapes of retinal images in the left and right eyes are the same, and a binocular fusion examination mode for examining whether there is fusion and suppression at long and short distances.

Also, the 3D image acquisition-examination mode may include at least one of a binocular disparity determination mode for determining a difference between visual powers in both eyes, a distance sensibility determination mode for determining an acquisition ability for distance perception, and a latent heterophoria determination mode for determining whether there is latent heterophoria.

In this case, the 3D examination test image may be an image stored in advance or an image received from the outside.

### BRIEF DESCRIPTION OF THE DRAWINGS

The above and/or other aspects will become more apparent from the detailed description of exemplary embodiments with reference to the accompanying drawings, in which:

FIG. 1 is a view illustrating a 3D image providing system according to an exemplary embodiment;

FIG. 2 is a block diagram illustrating a construction of a stereoscopic image display apparatus according to an exemplary embodiment;

FIGS. 3A and 3B are views for explaining a method for entering a 3D examination mode according to an exemplary embodiment;

FIGS. 4A to 4D are views for explaining a 3D examination test shape according to an exemplary embodiment;

FIGS. 5A to 5C are views for explaining a 3D examination test shape according to another exemplary embodiment;

FIGS. 6A to 6E are views illustrating 3D examination test figures according to various exemplary embodiments;

FIGS. 7A to 7D are views illustrating 3D examination test figures according to various exemplary embodiments;

FIGS. 8A and 8B are views illustrating an image providing method according to another embodiment; and

FIG. 9 is a flowchart for explaining a 3D image acquisition-examination method according to an exemplary embodiment.

### DETAILED DESCRIPTION OF EXEMPLARY EMBODIMENTS

Exemplary embodiments are described in greater detail below with reference to the accompanying drawings.

In the following description, like drawing reference numerals are used for the like elements, even in different drawings. The matters defined in the description, such as detailed construction and elements, are provided to assist in a comprehensive understanding of the invention. However, the described embodiments may be practiced without those specifically defined matters. Also, well-known functions or constructions are not described in detail since they would obscure the description with unnecessary detail.

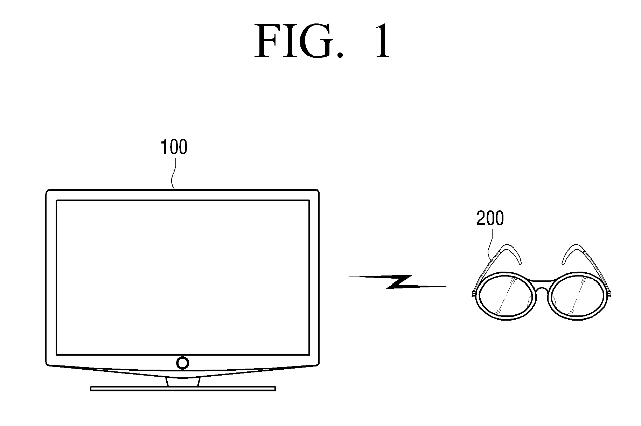

FIG. 1 is a view illustrating a 3D image providing system according to an exemplary embodiment. As illustrated in the drawing, the 3D image providing system includes a display apparatus 100 for displaying a 3D image on a screen, and 3D glasses 200 for watching the 3D image.

The display apparatus 100 is designed to display 3D images. Alternatively, display apparatus 100 may be designed to display both two dimensional (2D) images and 3D images.

When displaying the 2D image, the display apparatus 100 may use the same method as that of a conventional 2D display apparatus, and when displaying the 3D image, it may receive a 3D image output from a photographing device, such as a camera or the like. Alternately, the display apparatus 100 may receive a 3D image from a broadcasting station, after the image has been photographed by a camera and then edited/processed at the broadcasting station, process the received image, and then display the processed image on the screen. Particularly, the display apparatus 100 may process left and right eye images with reference to a format of the 3D image, to cause the images to be time-shared and alternatively displayed.

The 3D glasses 200 may be active type of shutter glasses. That is, a controller chip in the shutter glasses recognizes left/right image signals transmitted from the display apparatus 100 and rapidly opens and closes liquid crystals to transmit images through the liquid crystals or block the images, thereby generating a binocular disparity.

Particularly, the 3D glasses 200 may include an IR receiving unit (not shown) for receiving a synchronizing signal from the display apparatus 100.

The IR receiving unit may include an IR sensor part (not shown) to receive the synchronizing signal transmitted from the display apparatus 100, and a window part (not shown) to transmit the synchronizing signal therethrough to transmit it to the IR sensor part and to transmit outer light, such as a noise light or the like, therethrough in directions other than a direction toward the IR sensor part.

The 3D image providing system according to an exemplary embodiment may further include a camera (not shown) for producing the 3D image.

The camera is a type of photographing device for producing the 3D image. It produces a left eye image photographed to be provided to a left eye of a user, and a right eye image photographed to be provided to a right eye of the user. That is, the 3D image is made up of the left and right eye images, and these left and right eye images are alternatively provided to the left and right eyes of the user, respectively, thus generating a stereoscopic effect due to binocular disparity.

For this, the camera is made up of a left eye camera for producing the left eye image and a right eye camera for producing the right eye image, and a space between the left eye camera and the right eye camera is considered and determined based on an approximate distance between the left and right eyes of a human.

The camera transmits the photographed left and right eye images to the display apparatus 100. Particularly, the left and right eye images, which are transmitted to the display apparatus 100 from the camera, may be transmitted in a format in which a single frame consists of only one of the left eye image and the right eye image or a format in which a single frame consists of both the left eye image and the right eye image.

The camera may predetermine one of various formats of 3D images, and generate the 3D image according to the determined format to transmit to the display apparatus 100.

In the above description, 3D glasses 200 are explained as an indispensable component of glasses-type systems. However, embodiments described herein may be also applied to a display apparatus from which a 3D image can be viewed without the 3D glasses 200.

Hereinafter, a construction of the 3D glasses according to an exemplary embodiment will be explained in detail.

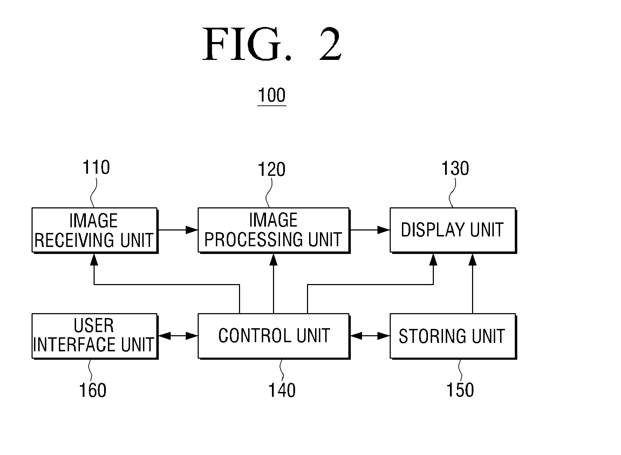

FIG. 2 is a block diagram illustrating a construction of the stereoscopic image display apparatus according to an exemplary embodiment.

Referring to FIG. 2, the display apparatus 100 includes an image receiving unit 110, an image processing unit 120, a display unit 130, a control unit 140, a storing unit 150, and a user interface unit 160.

The image receiving unit 110 receives and demodulates a 2D or 3D image signal, which is transmitted from a broadcasting station or a satellite by wire or wirelessly. Also, the image receiving unit 110 may be connected with an external appliance, such as a camera or the like, to receive a 3D image therefrom. The external appliance may be connected wirelessly, or by wire through an interface, such as a Separate Video (S-video) cable, a component cable, a composite cable, a D- subminiature (D-sub) cable, a digital visual interface (DVI) cable, a High-Definition Multimedia interface (HDMI™) cable, or the like. Since a 2D image processing method is known to those skilled in the art, an explanation will be made with priority given to a 3D image processing method hereinafter.

As described above, the 3D image is an image made up of at least one frame, such that both the left and right eye images may be included in a single frame. Alternately, each frame may include one of a left eye image and a right eye image, That is, the 3D image is an image formed according to one of various 3D formats.

Accordingly, the 3D image received into the image receiving unit 110 may be formed in various formats, particularly, a general top-bottom form, a side by side form, a horizontal interleave form, a vertical interleave form, a checker board form, or a sequential frame.

The image receiving unit 110 transmits the received 2D or 3D image to the image processing unit 120.

The image processing unit 120 performs tasks, such as signal processing, including video decoding, format analysis, video scaling and the like, and graphic user interface (GUI) addition, to the received 2D or 3D image.

Particularly, the image processing unit 120 produces a left eye image and a right eye image corresponding to a size of the screen (for instance, 1920 x 1080) by using the format of the 2D or 3D image input into the image receiving unit 110.

For instance, if the format of the 3D image is the general top-bottom form, the side by side form, the horizontal interleave form, the vertical interleave form, the checker board form, or the sequential frame form, the image processing unit 120 produces left and right eye images for displaying to a user by extracting left and right eye image portions from each image frame and magnification-scaling or interpolating the extracted left and right eye image portions.

Also, if the format of the 3D image is the general frame sequence form, the image processing unit 120 extracts left and right eye images from each image frame and prepares them for displaying to the user.

Information on the format of the input 3D image may be or not be included in the 3D image signal.

For instance, if the information on the format of the input 3D image is included in the 3D image signal, the image processing unit 120 analyzes the 3D image to extract the information on the format, and processes the received 3D image according to the extracted information. On the other hand, if the information on the format of the input 3D image is not included in the 3D image signal, the image processing unit 120 processes the received 3D image according to a format input by the user, or a format set up in advance.

The image processing unit 120 time-shares the extracted left and right eye images and alternately transmits them to the display unit 130. That is, the image processing unit 120 transmits the extracted left and right eye images to the display unit 130 in time order of" left eye image L1 → right eye image R1 → left eye image L2 → right eye image R2 → ....

The display unit 130 alternately outputs the left and right eye images output from the image processing unit 120 to provide them to the user.

In addition, the display unit 130 may display a 3D examination test shape for examining a degree of 3D image perception or acquisition according to a user command or a preset event. The preset event may include when there is an event for watching the 3D image (e.g., an input of a user command for watching the 3D image or the like is received), when the display apparatus 100 is turned on, when a predetermined time has elapsed after beginning to watch a 3D image, or another event.

The 3D examination test shape may include at least one of an image capable of being acquired by a left eye, an image capable of being acquired by a right eye, and an image capable of being simultaneously acquired by both the left eye and the right eye.

The 3D examination test shape may be one stored in advance or may be received from the outside through another communication method (for instance, via a local area network (LAN), or the internet, etc.).

The control unit 140 controls a general operation of the display apparatus 100 according to a user command received from the user interface unit 160 or a preset option.

The control unit 140 controls the image receiving unit 110 and the image processing unit 120 to receive the 3D image, to divide the received 3D image into the left eye image and the right eye image, and to scale or interpolate each of the divided left and right eye images in a size capable of being displayed in one picture.

In addition, the control unit 140 may control the display unit 130 to switch a polarization direction of the image provided through the display unit 130 to coincide with the switching of the left eye image and the right eye image.

The storing unit 150, which stores programs or the like needed to operate the display apparatus 100, may be embodied by a memory, a hard disk drive (HDD) or the like. For instance, the storing unit 150 may include a read only memory (ROM) storing a program for performing an operation of the control unit 140, and a random access memory (RAM) temporarily storing data generated as a result of the operation of the control unit 140. Also, the storing unit 150 may further include an electrically erasable and programmable ROM (EEROM) storing all sorts of reference data, and the like.

In addition, the storing unit 150 may store related data, such as a predetermined 3D examination test shape, user guide items according to a result of the examination, and the like.

The user interface unit 160 transmits a user command received from input means, such as a remote controller, an input panel, or the like, to the control unit 160.

Also, the user interface unit 160 functions to allow the user to input a degree of the user's acquisition of the 3D examination test shape.

In this case, the control unit 140 may control the display unit 130, an audio outputting unit (not shown) and the like to output a resultant value according to the degree of user's acquisition input through the user interface unit 160.

Here, the resultant value may be displayed in the form of an informing message including a 3D acquisition-examination result or a guide comment according to the degree of user's acquisition input through the user interface unit 160. Or, as occasion commands, it is possible for the resultant value to be displayed in the form of a sound or voice through the audio outputting unit.

Further, the control unit 140 may control to enter a 3D image acquisition-examination mode according to a user command or according to a preset event input through the user interface unit 160.

Here, the 3D image acquisition-examination mode may include an eyestrain acquisition-examination mode, and a 3D examination test shape for examining an eyestrain may include a predetermined magnitude of figure form with a midpoint capable of being perceived by one of the eyes, and a symbol form in the figure form capable of being perceived by the other of the eyes.

In addition, the 3D image acquisition-examination mode may include a definition acquisition-examination mode, and a 3D examination test shape for examining a definition comprises an upper figure form positioned on an upper side thereof to be capable of being perceived by one of the eyes, a lower figure form positioned on a lower side thereof to be capable of being perceived by the other of the eyes, and a line form positioned on a middle side thereof capable of being simultaneously perceived by both eyes.

Further, the 3D image acquisition-examination mode may include at least one of a coarse and fine examination mode for determining whether the user perceives a stereoscopic effect, a separate heterophoria examination mode for determining whether both eyes are rightly positioned to left and right image, respectively, a combined heterophoria examination mode for simultaneously examining a horizontal heterophoria and a vertical heterophoria, an aniseikonia-examination mode for examining whether magnitudes or shapes of retinal images in left and right eyes are the same, and a binocular fusion examination mode for examining whether there are a fusion and a suppression at long and short distances.

Also, the 3D image acquisition-examination mode may include at least one of a binocular disparity determination mode for determining a difference between visual powers in both eyes, a distance sensibility determination mode for determining an acquisition ability for distance perception, and a latent heterophoria determination mode for determining whether there is a latent heterophoria.

Here, the 3D examination test shape may be an image stored in advance or an image received from the outside.

Furthermore, the control unit 140 may control to display shapes practicable when the 3D examination test shape is incompletely acquired, according to the user command or a preset event input through the user interface unit 160. These shapes may be stored in advance or one received from the outside. Or, as occasion commands, they may be provided through a related server or the like.

The display apparatus 100 may further include an on screen display (OSD) processing unit (not shown) having a function of generating an OSD picture overlapped with and displayed on the 2D or 3D image output on display unit 130. Here, the OSD picture may provide menus, letters or figures, such as time or channel numbers, or the like on a display screen as overlapped with and displayed on the displayed image. For instance, as the user operates the input means, such as the input or operation panel or the remote controller, to select a desired function from the menus, a main menu, a submenu or the like may be displayed in an OSD form on the display screen. The menus may include optional items capable of being selected in the display apparatus, or items capable of adjusting functions of the display apparatus.

In addition, the OSD processing unit may perform tasks, such as a 2D/3D switching operations and operations for adjusting a transparency, color, size, shape, position, highlight effects, animation effects, and the like of the OSD picture, under a control of the control unit 140.

Also, the OSD processing unit may generate the OSD picture as a 2D or 3D picture according to a preset option, a preset event, or a user command.

The control unit 140 may control the OSD processing unit to adjust a transparency of the OSD picture when a 3D image is displayed through the display unit 130.

In addition, the control unit 140 may control the OSD processing unit to adjust the transparency of the OSD picture when at least one of the image and the OSD picture displayed through the display unit 130 is 3D.

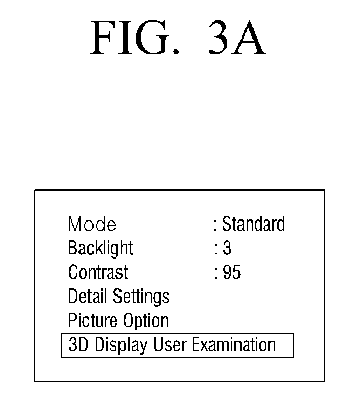

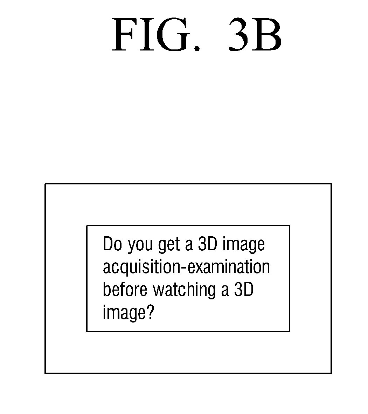

FIGS. 3A and 3B are views for explaining a method for entering a 3D examination mode according to an exemplary embodiment.

Referring to FIG. 3A, the user executes a 3D image user examination mode for examining a degree of her or his perception of the 3D image through the OSD menu. The relevant OSD menu may be displayed when the preset event is generated, or according to the user command. Here, the preset event may be before a 3D image watching mode begins, when a predetermined time elapses after beginning to watch the 3D image, when the relevant apparatus is turned on, or the like.

Referring to FIG. 3B, an inquiry window for inquiring about whether the 3D image user examination mode should be entered may be displayed, so that the user can execute the 3D image user examination mode by using the inquiry window. In this case, if there is a user command for watching the 3D image, the inquiry window may be displayed according to a preset option.

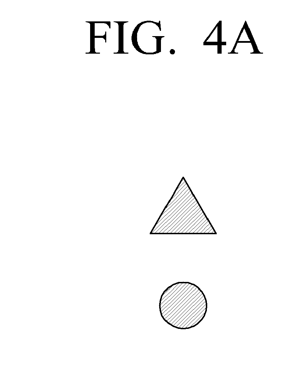

FIGS. 4A to 4D are views for explaining a 3D examination test shape according to an exemplary embodiment.

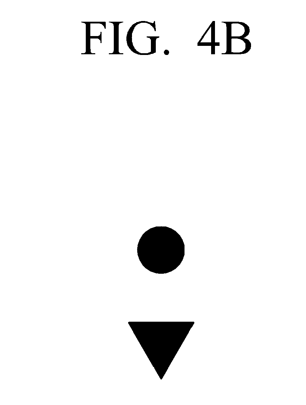

FIGS. 4A and 4B represent shapes capable of being seen by right and left eyes, respectively, when the user wears the 3D glasses.

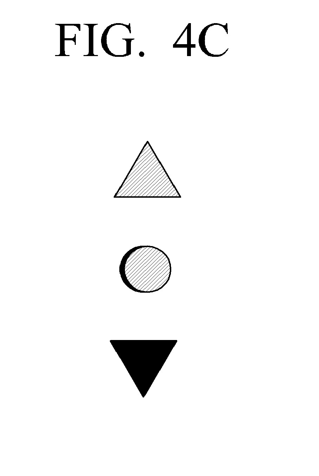

Also, FIG. 4C represent a shape, which as a shape displayed on the stereoscopic image display apparatus, can be seen by a human capable of perceiving stereoscopic vision.

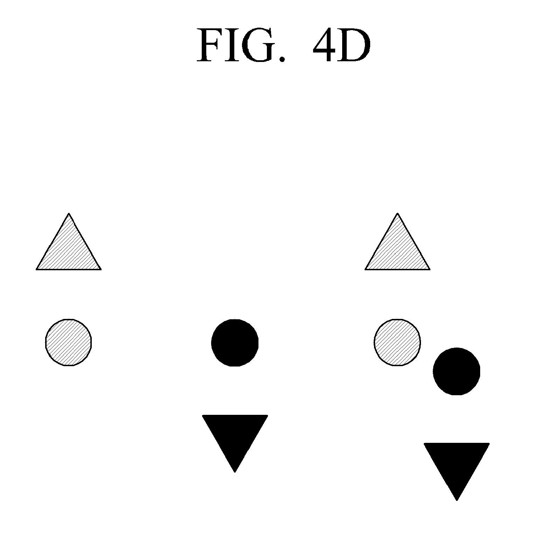

FIG. 4D represents shapes, which can be seen by a human not capable of perceiving stereoscopic vision.

That is, if the shapes displayed on the stereoscopic image display apparatus as illustrated in FIGS. 4A and 4B comes to be perceived as the shapes illustrated in FIG. 4D, it can be determined that a viewer does not perceive stereoscopic vision. If the shapes displayed as illustrated in FIGS. 4A and 4B are perceived as the shape illustrated in FIG. 4C, it can be determined that a viewer can perceive stereoscopic vision. Accordingly, the viewer can judge by herself or himself whether she or he can accurately perceive the 3D image, and also comes to be able to see a degree of other people's acquisition to the 3D image.

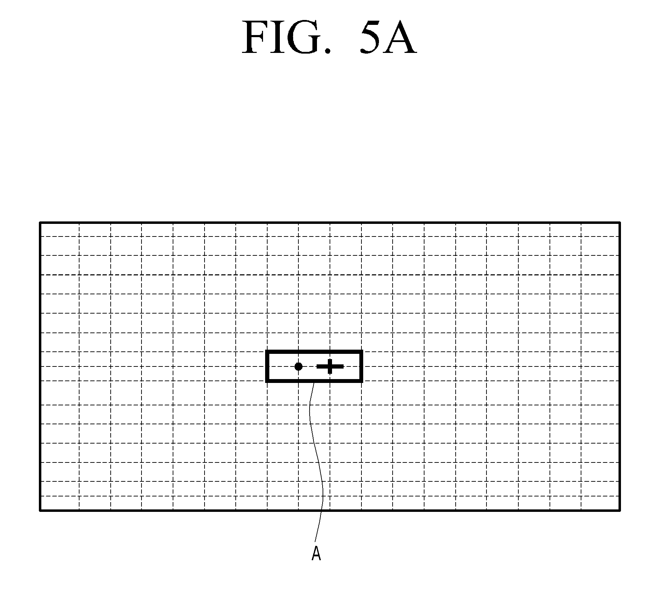

FIGS. 5A to 5C are views for explaining a 3D examination test shape according to another exemplary embodiment.

To continuously maintain perception of a 3D visual image while watching a 3D display, the angle of focus of the eyes should be accurately coincided with each other. To comfortably maintain a coincidence between the angle of focus of the eyes , the angle of focus of the eyes should coincide both in a relaxed state, and when focused at a TV watching distance.

Accordingly, the 3D image acquisition-examination mode according to an exemplary embodiment may include an eyestrain acquisition-examination mode, and a 3D examination test shape for examining eyestrain may include a predetermined figure form with a midpoint capable of being acquired by one eye, and a symbol form in the figure form capable of being acquired by the other eye. FIGS. 5A to 5C illustrate a 3D examination test shape for examining the eyestrain, which can be provided in the eyestrain acquisition-examination mode according to an exemplary embodiment.

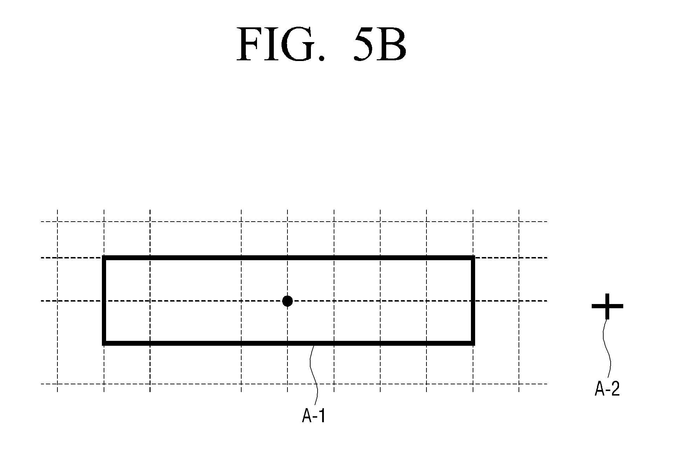

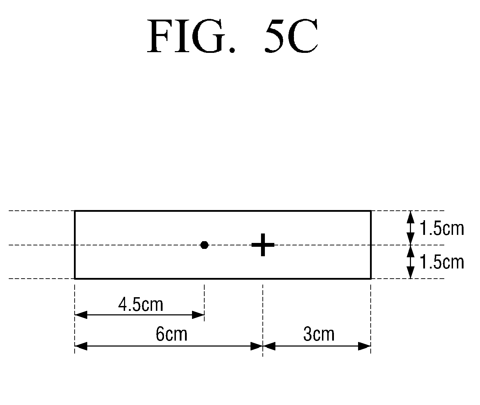

The eyestrain 3D examination test shape illustrated in FIG. 5A may include a rectangular form A-1 with a midpoint in the form of a dot capable of being acquired by one eye, and a `+' form A-2 capable of being acquired by the other eye, as illustrated in FIG. 5B. On the other hand, at a typical TV watching distance of about 3 m, the 3D examination test shape may appear to a viewer as shown in FIG. 5C. The particular dimensions of the shape or sign may be changed according to a design of those skilled in the art. For instance, the dimension of the sign or symbol can be designed, so that the size thereof is perceived as changing according to distance and, for example, is proportional to the watching distance.

Additionally, a degree of eyestrain of the user may be determined according to a position of the sign with respect to the midpoint of the rectangular shape in an image, as perceived by the user.

To be specific, if the user perceives the shape displayed as illustrated in FIG. 5C on a TV screen while wearing the 3D glasses, if the '+' sign is seen close to the midpoint in the rectangular form, it can be determined that she or he feels little eyestrain and has less discomfort while watching. If the `+' sign is seen far away from the rectangular shape, it can be determined that the user has a high level of eyestrain. At this time, it can be advised that the user watches the 3D TV after obtaining glasses prescribed by experts.

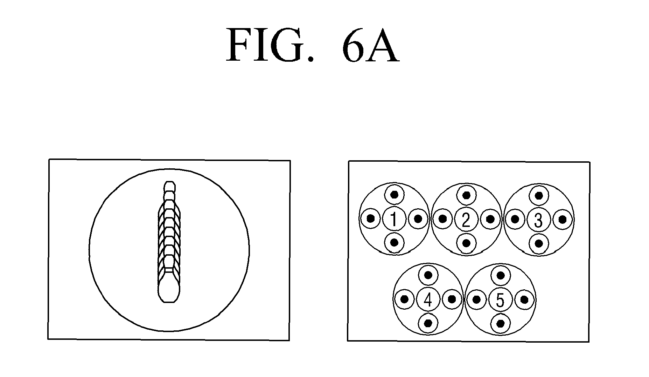

FIGS. 6A to 6E are views illustrating 3D examination test figures according to various exemplary embodiments of the present invention.

A shape as shown in FIG. 6A may be a 3D examination test shape used for a coarse and fine examination mode to determine whether the user perceives the 3D effect before trying to get a precise examination for binocular vision.

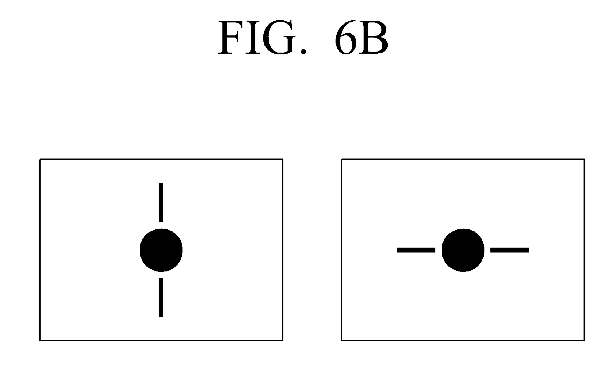

A shape as shown in FIG. 6B may be a 3D examination test shape for determining whether the eyes are appropriately positioned for the left and right images, respectively.

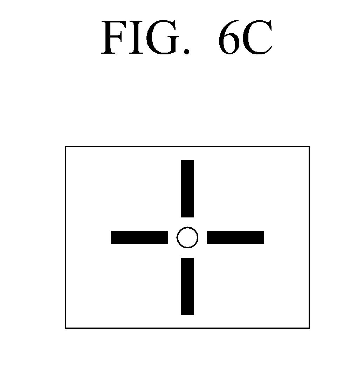

A shape as shown in FIG. 6C may be a 3D examination test shape for simultaneously examining horizontal heterophoria and vertical heterophoria.

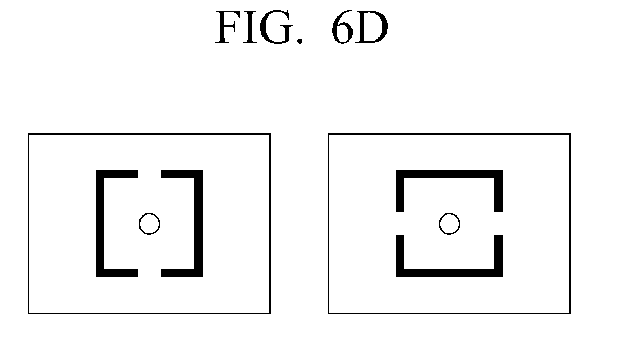

A shape as shown in FIG. 6D may be a 3D examination test shape for examining a state in which magnitudes or shapes of retinal images in the left and the right eyes are not the same.

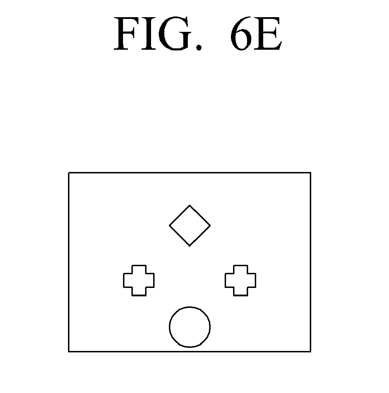

A shape as shown in FIG. 6E may be a 3D examination test shape for examining whether there is fusion and suppression at long and short distances.

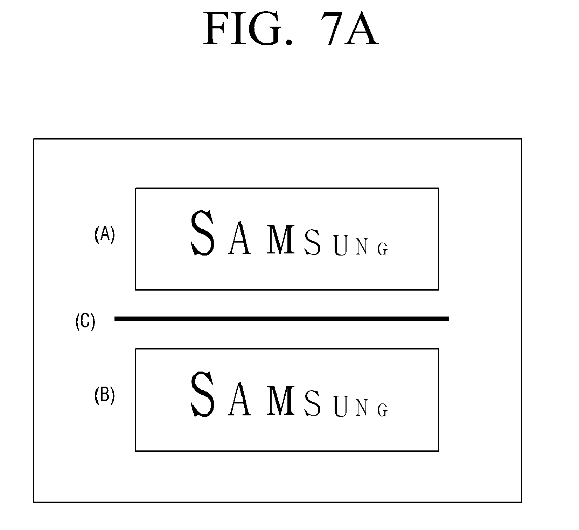

FIGS. 7A to 7D are views illustrating 3D examination test figures according to various exemplary embodiments.

As illustrated in FIGS. 7A to 7D, images, which are capable of examining visual characteristics of the user, may be provided.

A 3D examination test shape as shown in FIG. 7A, as a shape capable of being provided for use in a definition acquisition-examination mode for determining whether the both eyes perceive the same sharpness of focus, may include an upper figure form positioned on an upper side and capable of being viewed by one eye, a lower figure form positioned on a lower side and capable of being viewed by the other eye, and a line form positioned in the middle side and capable of being simultaneously viewed by both eyes.

As illustrated in FIG. 7A, the 3D examination test shape for examining the definition of the both eyes may be made up of an upper figure portion A capable of being viewed by one eye, a lower figure portion B capable of being viewed by the other eye, and a middle figure portion C capable of being simultaneously viewed by both eyes.

If a user is able to correctly perceive the 3D effect and the sharpness of focus is superior, the user will view, with the same degree of sharp focus, the upper figure portion A capable of being seen by the right eye for example, and the lower figure portion B capable of being seen by the left eye for example.

If there is an abnormality in the user's perception of the 3D effect and the focus is not sharp, the user won't see one or both of the upper figure portion A and the lower figure portion B with equal sharpness, and one or both figure portions may be unfocused.

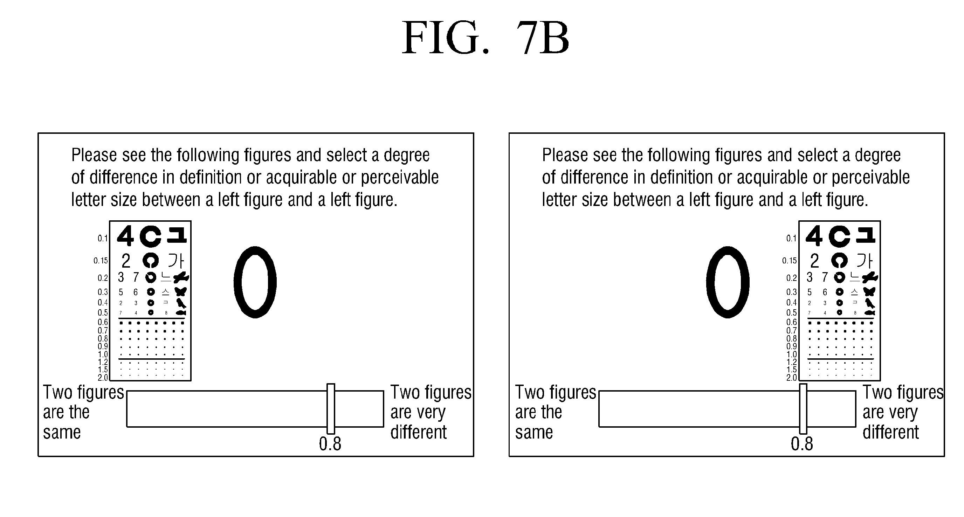

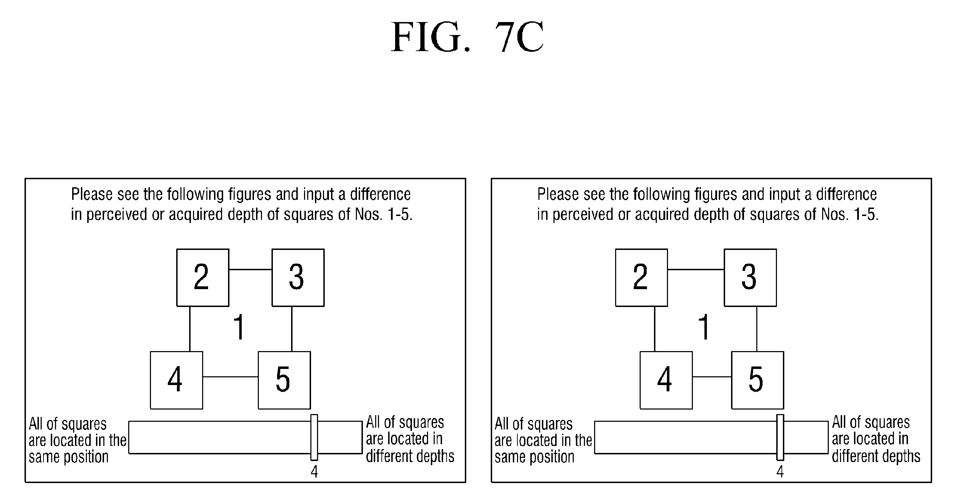

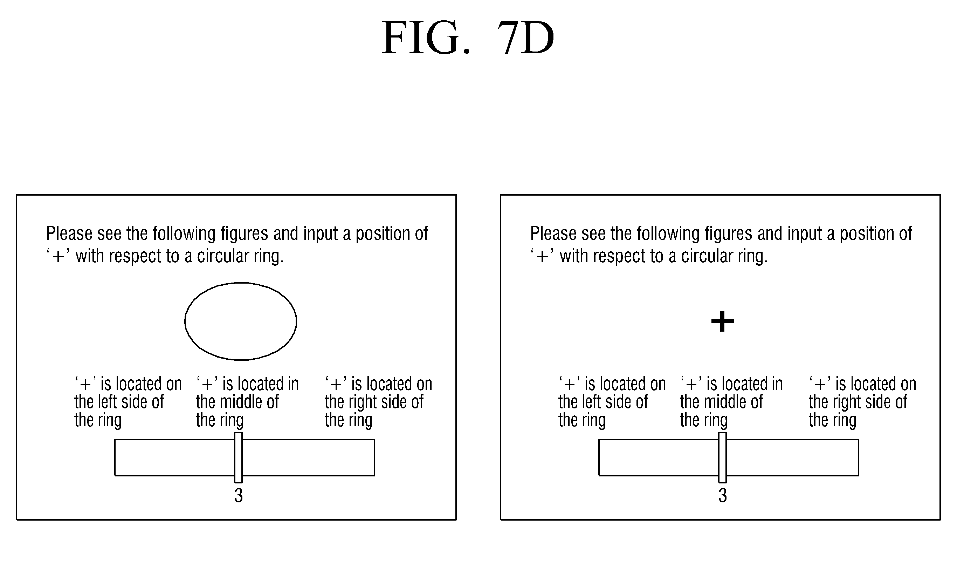

A 3D examination test shape as shown in FIG. 7B is a test shape for determining a difference between visual powers of the eyes, a 3D examination test shape as shown in FIG. 7C is a test shape for determining an acquisition ability for distance perception, and a 3D examination test shape as shown in FIG. 7D is a test shape for determining a latent cross-eye (heterophoria). According to these, the images, which are capable of use in examining the visual characteristics, such as an astigmatism, the heterophoria, cross-eye, binocular disparity, a lack of the acquisition ability for distance perception, and the like, which cause side effects from watching a 3D image, are provided, so that a degree of perception of the images is determined and input, and utilized.

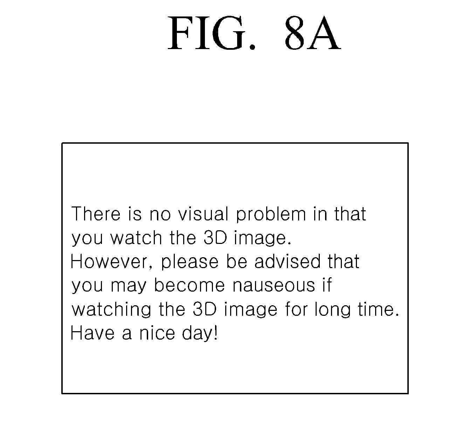

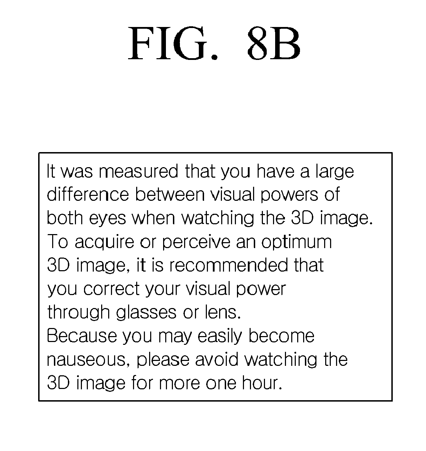

FIGS. 8A and 8B are views illustrating an image providing method according to another exemplary embodiment.

A degree of user's acquisition to the 3D examination test shapes according to the various exemplary embodiments of the present invention as described above may be input through a user interface (UI).

After that, the input degree of user's acquisition may be synthesized to inform the user of what is important about the visual characteristics. To be specific, the input content is analyzed to inform the user of a requirement, a degree of danger, a proposed watching method, etc. As occasion demands, the user can improve his or her 3D watching experience by a method, such as the addition of a prism lens to the 3D glasses.

FIGS. 8A and 8B illustrate examples of information, which can be provided to the user.

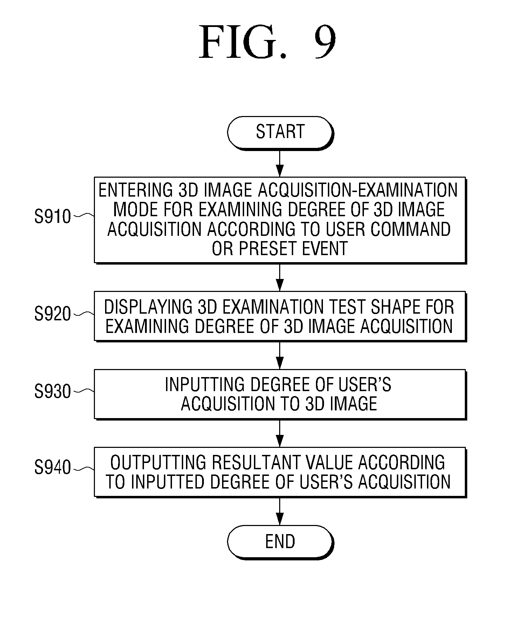

FIG. 9 is a flowchart for explaining a 3D image acquisition-examination method according to an exemplary embodiment.

According to the 3D image acquisition-examination method as illustrated in FIG. 9, if a 3D image acquisition-examination mode for examining a degree of 3D image acquisition is entered according to a user command or a preset event (S910), a 3D examination test shape is displayed (S920).

Subsequently, a degree of user's acquisition of the 3D examination test shape is input (S930), and a resultant value according to the input degree of user's acquisition is output (S940).

Here, the output of the resultant value may include the display of an informing message including a 3D acquisition-examination result or a guide comment according to the input degree of user's acquisition.

In this case, the 3D examination test shape may include at least one of an image capable of being viewed by the left eye, an image capable of being viewed by the right eye, and an image capable of being simultaneously viewed by both the left eye and the right eye.

In addition, shapes practicable if the 3D examination test image is incompletely acquired may be displayed according to a user command or a preset event.

Further, the 3D image acquisition-examination mode may include an eyestrain acquisition-examination mode, and a 3D examination test shape for examining an eyestrain may include a predetermined figure form with a midpoint capable of being viewed by one eye, and a symbol form in the figure form capable of being viewed by the other eye.

Here, the 3D image acquisition-examination mode may include a focus acquisition-examination mode, and a 3D examination test shape for examining a focus may include an upper figure form positioned on an upper side thereof and capable of being viewed by one eye, a lower figure form positioned on a lower side thereof and capable of being viewed by the other eye, and a line form positioned between the lower and upper figure forms and capable of being simultaneously viewed by both eyes.

Moreover, the 3D image acquisition-examination mode may include at least one of a coarse and fine examination mode for determining whether the user perceives the three-dimensional effect, a separate heterophoria examination mode for determining whether the eyes are correctly positioned with respect to each other in a horizontal (left and right) direction, a combined heterophoria examination mode for simultaneously examining a horizontal heterophoria and a vertical heterophoria, an aniseikonia-examination mode for examining whether magnitudes or shapes of retinal images in the left and the right eyes are the same, and a binocular fusion examination mode for examining whether there is fusion and suppression at long and short distances.

Also, the 3D image acquisition-examination mode may include at least one of a binocular disparity determination mode for determining a difference between visual powers in the both eyes, a distance sensibility determination mode for determining an acquisition ability for distance perception, and a latent heterophoria determination mode for determining whether there is a latent heterophoria.

In this case, the 3D examination test shape may be an image stored in advance or an image received from the outside.

According to this, the user can examine the degree of acquisition of the 3D image by herself or himself, and obtain various information according to the examination results. Also, the user comes to be able to see the degree of other people's 3D image acquisition. Accordingly, the 3D watching environment can be improved.

The foregoing exemplary embodiments and advantages are merely exemplary and are not to be construed as limiting. The described embodiments can be readily applied to other types of apparatuses. Also, the description of the exemplary embodiments is intended to be illustrative, and not to limit the scope of the claims, and many alternatives, modifications, and variations will be apparent to those skilled in the art.

## Claims

1. A display apparatus, providing a three-dimensional (3D) image acquisition-examination mode, comprising:
a display unit (130) to display a 3D examination test shape for examining a degree of 3D image acquisition;
a user interface unit (160) to allow a user to input a degree of the user's acquisition of the 3D examination test shape; and
a control unit (140) controlling to output an indication according to the degree of user's acquisition inputted through the user interface unit.

2. The apparatus of claim 1, wherein the control unit is arranged to control the display unit to display an informing message including a 3D acquisition-examination result or a guide comment according to the degree of user's acquisition inputted through the user interface unit.

3. The apparatus of claim 1 or 2, wherein the control unit is arranged to control to enter the 3D image acquisition-examination mode according to a user command inputted through the user interface unit or a preset event.

4. The apparatus of any one of claims 1 to 3, wherein the 3D examination test shape comprises an image capable of being viewed by a left eye, an image capable of being viewed by a right eye, and an image capable of being simultaneously viewed by both the left eye and the right eye.

5. The apparatus of any one of claims 1 to 4, wherein the control unit is arranged to control to display shapes practicable when the 3D examination test shape is incompletely acquired, according to a user command inputted through the user interface unit or a preset event.

6. The apparatus of any one of claims 1 to 5, wherein the 3D image acquisition-examination mode comprises an eyestrain acquisition-examination mode, and the 3D examination test shape comprises a predetermined figure form, a midpoint capable of being viewed by one of the user's eyes, and a symbol form in the figure form capable of being viewed by the other of the user's eyes.

7. The apparatus of any one of claims 1 to 6, wherein the 3D image acquisition-examination mode comprises a definition acquisition-examination mode, and the 3D examination test shape comprises an upper figure form capable of being viewed by one of the user's eyes, a lower figure form positioned below the upper figure form and capable of being viewed by the other of the user's eyes, and a line form positioned between the upper figure form and the lower figure form and capable of being simultaneously viewed by both the user's eyes.

8. The apparatus of any one of claims 1 to 7, wherein the 3D image acquisition-examination mode comprises at least one of a coarse and fine examination mode for determining whether the user perceives a three-dimensional effect, a separate heterophoria examination mode for determining whether the user's eyes are correctly positioned with respect to a horizontal direction, a combined heterophoria examination mode for simultaneously examining a horizontal heterophoria and a vertical heterophoria, an aniseikonia examination mode for examining whether magnitudes or shapes of retinal images in the user's left and right eyes are the same, and a binocular fusion examination mode for examining whether there are fusion and suppression at long and short distances.

9. The apparatus of any one of claims 1 to 8, wherein the 3D image acquisition-examination mode comprises at least one of a binocular disparity determination mode for determining a difference between visual powers in the user's eyes, a distance sensibility determination mode for determining an acquisition ability for distance perception, and a latent heterophoria determination mode for determining whether there is a latent heterophoria.

10. The apparatus of any one of claims 1 to 9, wherein the 3D examination test shape comprises an image stored in advance or an image received from an external source.

11. A three-dimensional (3D) image acquisition-examination method providing a 3D image acquisition-examination mode comprising:
displaying a 3D examination test shape for examining a degree of 3D image acquisition (S920);
inputting a degree of a user's acquisition of the 3D examination test shape (S930); and
outputting an indication according to the inputted degree of user's acquisition (S940).

12. The method of claim 11, wherein the outputting the indication comprises displaying an informing message including a 3D acquisition-examination result or a guide comment according to the inputted degree of user's acquisition.

13. The method of claim 11 or 12, further comprising entering the 3D image acquisition-examination mode according to a user command or a preset event.

14. The method of any one of claims 11 to 13, wherein the 3D examination test shape comprises at least one of an image capable of being viewed by a left eye, an image capable of being viewed by a right eye, and an image capable of being simultaneously viewed by both the left eye and the right eye.

15. The method of any one of claims 11 to 14, further comprising displaying shapes practicable when the 3D examination test shape is incompletely viewed , according to a user command or a preset event.
